# EUROPEAN PATENT APPLICATION

(11) **EP 1 508 624 A1**
(43) Date of publication of application: **23.02.2005**
(21) Application number: 03292082.9
(22) Date of filing: 22.08.2003
(51) Int. Cl.: C12Q 1/70, C12Q 1/68

(54) **A quantification method for integrated viruses**

(71) Applicant: INSTITUT NATIONAL DE LA SANTE ET DE LA RECHERCHE MEDICALE (INSERM), 75013 Paris (FR)
(72) Inventor: Sonigo, Pierre, 75015 Paris (FR); Brussel, Audrey, 92190 Meudon (FR); Delelis, Olivier, 78220 Viroflay (FR); Leste-Lasserre, Thierry, 33640 Ayguemorte Les Graves (FR); Pierre-Broche, Sophie, 78400 Chatou (FR); Petit, Caroline, 75015 Paris (FR)
(74) Representative: Bernasconi, Jean

(57) **Abstract**

The invention relates to a method of detecting and/or quantifying a virus, such as HIV, integrated in a cellular genome, and to kits useful to perform this method. This two-step method calls for an anchored viral primer and *Alu* primers for a first round of PCR (*Alu* PCR) and a primer for the anchor along with another viral primer for the second round of PCR ("nested PCR").

## Description

The present invention relates to a method for detecting and/or quantifying a virus integrated in a cellular genome.

The RNA genome of Human Immunodeficiency virus type-1 (HIV-1) is, like other retroviruses, reverse transcribed in the cytoplasm into a double stranded linear DNA containing a copy of the Long Terminal Repeats (LTR) at each terminus. The resulting linear DNA molecule moves into the nucleus as a component of the preintegration complex, where it may integrate into the host cell genome. This process is dependent on the viral integrase activity, which is essential for a productive infection. In addition to linear DNA species, HIV-1 DNA circles with one or two LTR are detected in the nucleus.

While the unintegrated forms of HIV-1 DNA can be readily detected by using standard polymerase chain reaction (PCR) or Southern hybridization techniques, quantitative analysis of integrated HIV-1 DNA has been hampered by the lack of a reliable assay. Early studies have attempted to quantify integrated viral DNA by one-step real-time Alu-LTR PCR using one primer annealing in the HIV-1 LTR and a second in the highly repeated chromosomal *Alu* element (*Alu*-LTR PCR) (Butler et al, 2001; Butler et al, 2002, and international patent application WO01/81541). *Alu* repeated sequences are interspersed in the human genome and number at least 900,000 copies per haploid genome, giving an average distance of 4 kilobases between *Alu* elements (Britten *et al.* 1988). However, these prior Alu-LTR PCR methods have limited sensitivity and do not take into account that the distance between an integrated viral DNA and its nearest *Alu* repeat is variable. Two-step nested PCR methods, which used one primer annealing either in the HIV gag sequence (O'Doherty *et al.,* 2002) or in HIV LTR region (Chun *et al.,* 1997) and a second in the *Alu* element, were also proposed.

During PCR, the target sequence is amplified with an exponential rate until exhaustion of one of the PCR reagents and plateauing of the amplification. At any cycle of the exponential phase of a PCR amplification, the copy number of a target sequence is thus quantitatively related to that of PCR products. The higher the number of target sequence is, the sooner exhaustion of PCR reagents occurs. Accordingly, determination of target sequence copy number is to be performed during the PCR exponential phase. The dynamic range of a quantification assay is the range between the highest and the lowest copy number of a target sequence that can be quantified accurately.

The O'Doherty and Chun methods did not prevent the second-round amplification of non-preamplified HIV DNA and the dynamic range of analysis was less extensive than the one obtained according to the method of the present invention.

Therefore there was still a need for a simple and accurate method for detecting and quantifying integrated HIV DNA, and more generally DNA of any virus integrated in cellular genomes.

The inventors have developed a new method that fulfills this need. This is a highly sensitive two-step method, based on *Alu-* nested PCR that makes use of an anchor sequence. More particularly this two-step method calls for an anchored viral primer and *Alu* primers for a first round of PCR (*Alu* PCR) and a primer for the anchor along with another viral primer for the second round of the PCR ("nested PCR").

In one aspect of the invention, there is provided a PCR method to detect or quantify integrated forms of a virus in cellular chromosomal DNA.

In another aspect of the invention, there is provided a kit for performing this method.

This method allows accurate assessment of the frequency of productively and latently infected cells together in patients.

Another subject of the invention is thus an *in vitro* method for monitoring the efficacy of a vaccine or of a therapeutic treatment, against a virus infection in a human or animal, which method comprises determining whether the virus is integrated in the cellular genome of the human or animal, by means of the detection and/or quantification method described herein, wherein the fewer copies of integrated virus are detected in the cellular genome, the more efficient the vaccine or therapeutic treatment is.

Such method is especially useful for *in vitro* screening of compounds for their ability to inhibit the integration of a virus.

A further subject of the invention is an *in vitro* method for verifying the safety of an attenuated living viral vaccine in a human or animal, which method comprises determining whether the attenuated virus is integrated in the cellular genome of the human or animal, by means of the detection and/or quantification method described herein, wherein the fewer copies of the attenuated virus are detected in the cellular genome, the safer the viral vaccine is.

A further subject of the invention is an *in vitro* method for assessing the transfer efficacy of a viral vector useful in gene therapy in a human or animal, which method comprises detecting and/or quantifying the viral vector integrated in the cellular genome by means of the detection and/or quantification method described herein, wherein the greater copies of the viral vector are detected in the cellular genome, the more efficient the transfer is.

A still further subject of the invention is an *in vitro* method for assessing the risk of cellular gene disruption caused by the transfer of a viral vector useful in gene therapy in a human or animal, which method comprises detecting and/or quantifying the viral vector integrated in the cellular genome of the human or animals by means of the detection and/or quantification method described herein, wherein the fewer copies of the viral vector are detected in the cellular genome, the lower the risk of cellular gene disruption is.

These methods, especially the methods for assessing the risk of cellular disruption and the transfer efficacy of a viral vector intended for gene therapy, may be performed either on a biological sample from a human or animal, or on cell cultures, before any preclinical or clinical trials.

### The method of the invention

The present invention thus provides an *in vitro* method of detecting and/or quantifying a virus that is integrated in a cellular genome, which method comprises the steps of:
(a) amplifying DNA of a biological sample with PCR using at least one primer for an Alu sequence and a primer for a viral sequence, wherein the primer for the viral sequence is linked to an anchor sequence that is not capable of hybridising to the viral genome nor to the cellular genome;
(b) subjecting the amplified DNA to another amplification with PCR using a primer for the viral sequence that was amplified in step (a), and a primer for the anchor sequence that was amplified in step (a);
(c) detecting and/or quantifying the DNA that was amplified through steps (a) and (b), and that is correlated with the number of copies of the virus integrated in the cellular genome.

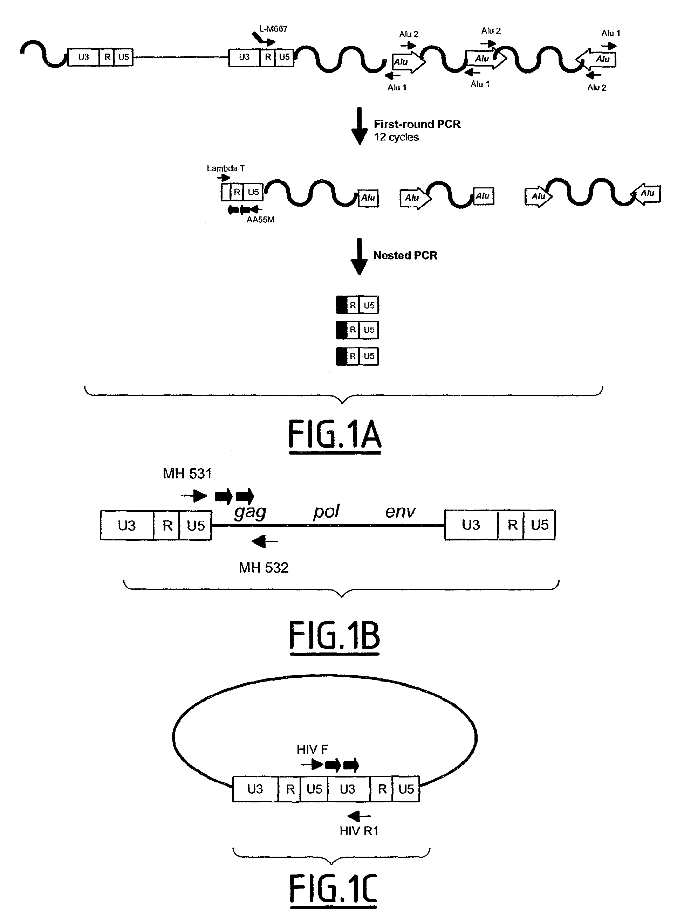

A schematic drawing that illustrates this method is set forth in Figure 1A.

Determination of target sequence copy number in such two-step PCR assay, requires that the first-round PCR is stopped during the exponential phase of the reaction. The higher the number of cycles performed during the first-round PCR is, the narrower the dynamic range of the quantification assay is. However a sufficient number of cycles is required to obtain enough sensitivity.

Step (a) thus preferably comprises from 10 to 15 cycles of PCR, more preferably 12 cycles of PCR. This number of cycles indeed led to the most satisfying results in terms of sensitivity and offered the greatest dynamic range of analysis.

In the same prospect, it may be useful to perform a dilution, such as a ten-fold dilution between step (a) and step (b).

In a preferred embodiment, the first-round PCR cycle conditions (*i*.*e*. step (a)) are as follows: a denaturation step of 8 min at 95 °C and then 12 cycles of amplification (95 °C 10 s, 60 °C 10 s , 72 °C 170 s).

Preferably, step (b) may begin with a denaturation step (95 °C for 8 min) followed by 50 cycles of amplification (95 °C 10 s, 60 °C 10 s , 72 °C 9 s).

In a particular embodiment of the invention, a purification step is provided between step (a) and step (b), which purification step increases the selectivity of the method. This purification step comprises purifying the amplified DNA from step (a) that has incorporated an Alu primer, so that the amplification of step (b) occurs only on this purified DNA

The cellular genome may be of any organism susceptible to an infection by a virus, especially a retrovirus. Humans are the preferred target, but the present invention also encompasses detecting or quantifying a virus integrated in the cellular genome of other non-human animals, such as mammals, including rodents, monkeys, feline, dogs, horses, goats, cattle, sheep, or porcine.

The biological samples are any tissue or cell sample or biological fluid that contains cellular chromosomal DNA. Advantageously it can be blood, sections of lymph nodes or lymphocytes. Noteworthy the method of the invention can be performed on total DNA, i.e. without any need for first extracting the genomic DNA. The method of the invention can also be performed on crude samples, *e.g.* total blood, without extensive purification of the DNA therefrom.

In a particular embodiment the method comprises the steps of:
(a) amplifying DNA of a human biological sample with 12 cycles of PCR using two outward-facing primers for *Alu* sequences and a primer for a HIV viral sequence, wherein the primer for the viral sequence is SEQ ID NO:2, linked to an anchor sequence SEQ ID NO:1, so that the sequence of the anchored primer consists of SEQ ID NO:3;
(b) subjecting the amplified DNA to a ten-fold dilution followed by another amplification with PCR using a primer of SEQ ID NO: 4 for the viral sequence that was amplified in step (a), and a primer of SEQ ID NO: 1 for the anchor sequence that was amplified in step (a) ; in the presence of probes of SEQ ID NO:7 and SEQ ID NO:8;
(c) detecting and/or quantifying the DNA that was amplified through steps (a) and (b), and that is correlated with the number of copies of the HIV virus that is integrated in the human genome.

### General definitions

In accordance with the present invention there may be employed conventional molecular biology, microbiology, and recombinant DNA techniques within the skill of the art. Such techniques are explained fully in the literature. See, e.g., Sambrook et al., 1989 ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)].

A "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded helix. The term nucleic acid molecule, and in particular DNA or RNA molecule, refers only to the primary and secondary structure of the molecule, and does not limit it to any particular tertiary forms. Thus, this term includes double-stranded DNA round, *inter alia,* in linear or circular DNA molecules, plasmids, and chromosomes. Unless otherwise specified, sequences are described according to the normal convention of giving only the sequence in the 5' to 3' direction along the nontranscribed strand of DNA (i.e., the strand having a sequence homologous to the mRNA).

As used herein, the term "oligonucleotide" refers to a nucleic acid, generally of at least 10, preferably at least 15, and more preferably at least 20 nucleotides, preferably no more than 100 nucleotides, more preferably no more than 40 nucleotides that is hybridizable to a genomic DNA molecule, or other nucleic acid of interest.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (see Sambrook et al., 1989). The conditions of temperature and ionic strength determine the "stringency" of the hybridization. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., 1989, 9.50-9.51). For hybridization with shorter nucleic acids, i.e., oligonucleotides, the position of mismatches becomes more important, and the length of the oligonucleotide determines its specificity (see Sambrook et al., 1989, 11.7-11.8). A minimum length for a hybridizable nucleic acid is at least about 10 nucleotides, preferably at least about 15 nucleotides, and more preferably the length is at least about 20 nucleotides.

In a specific embodiment, the term "standard hybridization conditions" refers to a Tm of 55°C, and utilizes conditions as set forth above. In a preferred embodiment, the Tm is 60°C. In a more preferred embodiment, the Tm is 65°C. In a specific embodiment, "high stringency" refers to hybridization and/or washing conditions at 68°C in 0.2 X SSC (SCC is a 0.15 M NaCl, 0.015 M Na-citrate) at 42°C in 50 % formamide, 4 X SSC or under conditions that afford levels of hybridization equivalent to those observed under either of these two conditions.

The term "not capable of hybridizing", when referring to the anchor sequence that is not capable of hybridising to the viral genome nor to the cellular genome, means that the anchor sequence shows no identity nor homology on its full length with the viral genome nor the cellular genome, or shows an identity (on its full length) of less than 20%, preferably less than 15%, most preferably less than 10% with the viral genome nor the cellular genome. It is therefore unable to anneal to the viral genome nor to the cellular genome under standard hybridisation conditions.

"Amplification" of DNA as used herein denotes the increase in the concentration of a particular DNA sequence within a mixture of DNA sequences. The use of polymerase chain reaction (PCR) is more particularly contemplated in the context of the invention.

As used herein, the terms "primer" and "probe" refer to the function of the oligonucleotide. A primer is an oligonucleotide used for amplifying a target sequence typically by extension of the oligonucleotide after hybridization to the target sequence or by ligation of multiple oligonucleotides which are adjacent when hybridized to the target sequence. A probe is an oligonucleotide that is used to capture or detect a target sequence to which it hybridizes.

An *"Alu* primer" or "a primer for an *Alu* sequence" is a primer that is capable of specifically hybridising to an *Alu* sequence present in the cellular genome of the host under standard conditions of hybridisation.

A "viral primer" or "a primer for a viral sequence" is a primer that is capable of specifically hybridizing to a region of the viral sequence , under standard conditions of hybridization.

Similarly the "primer for the anchor sequence that was amplified in step (a)" is a primer that is capable of specifically hybridizing to the sequence resulting from the amplification of the anchor sequence, under standard conditions of hybridization. Preferably this primer has the same sequence as the anchor sequence.

A "nested" PCR means that two pairs of PCR primers are used for a single locus. The first pair amplifies the locus as seen in any PCR experiment. The second pair of primers ("nested primers") bind within the first PCR product and produce a second PCR product that will be shorter than the first one. The logic behind this strategy is to increase detection sensitivity. Moreover if the wrong locus were amplified by mistake, the probability is very low that it would also be amplified a second time by a second pair of primers.

### The anchor

The anchor sequence is a nucleic acid sequence selected so that it is not capable of hybridising to the viral genome nor to the cellular *(e.g. human)* genome, especially under the conditions of stringency used for the step(a) of the PCR method of the invention.

In the present description it is also referred to as the "heel sequence", insofar as it extends the end of the viral primer .The anchor sequence is preferably between 15 and 25 nucleotide-long. This sequence may advantageously be a sequence selected from the genome of a Lambda phage, such as SEQ ID NO:1.

The primer for the anchor sequence that was amplified in step (a) may consist of SEQ ID NO:1, too.

### The viral primers

The method of the invention allows for the detection and/or quantification of any virus that is capable of integrating the cellular genome. According to a preferred embodiment, the virus is a retrovirus, preferably HIV (Human Immunodeficiency virus) or FIV (Feline Immunodeficiency Virus), SIV (Simian Immunodeficiency Virus), or more generally vectors encoding retrovirus-derived sequences useful for gene transfer and gene therapy, such as MLV (Moloney Leukemia Virus), or HFV (Human Foamy Virus).

Hepatitis virus (that is an adenovirus) is known to integrate the cellular genome too, and may also be detected or quantified by means of the method of the invention.

The primer for the viral sequence used in step (a) is selected within one of the end regions of the integrated virus so that the polymerase used for the PCR is capable of extending the strain, from this primer to include the junction with the cellular genome and an *Alu* sequence of the cellular genome.

The term "end regions of the integrated virus" means the sequence of the virus close to the cellular genome wherein it is integrated. For example, when the virus is a retrovirus, the primer sequence used in step (a) preferably hybridises to an LTR sequence of the retrovirus.

In a preferred embodiment the primer for the viral sequence used in step (a) hybridises to the R region of the 3'LTR of the retrovirus (e.g., HIV), and the primer used in step (b), for the viral sequence amplified in step (a), is an antisense primer that hybridises to the U5 region of the 3'LTR of the retrovirus, as depicted in Fig. 1A.

In another embodiment, the primer for the viral sequence used in step (a) hybridises to the R region of the 5'LTR of the retrovirus (e.g., HIV), and the primer used in step (b), for the viral sequence amplified in step (a), is an antisense primer that hybridises to the U3 region of the 5'LTR of the HIV.

In a particular embodiment of the method for detecting or quantifying HIV, the primer for the viral sequence used in step (a) comprises SEQ ID NO:2. The anchored primer for the viral sequence extended at the '5 terminus with the anchor sequence of SEQ ID NO:1 has then the sequence SEQ ID NO:3.

The primer used in step (b), for the viral sequence amplified in step (a), is selected so that the PCR of step (a) is "nested". For that purpose, the primer used in step (b), for the viral sequence amplified in step (a) specifically hybridises to a motif sequence within the viral amplification product resulting from step (a).

For instance, when the target virus is HIV, the viral primer used in step (b) may consist of SEQ ID NO:4.

### The Alu primers

Although it is possible to perform the method of the invention with one primer for the *Alu* sequence, it is advantageous for the PCR of step (a) to employ two outward-facing primers for the *Alu* sequences. The use of two outward-facing *Alu* primers optimizes the probability to amplify a viral sequence, as *Alu* elements are present in either orientation relative to the integrated virus, as depicted in Figure 1A.

For instance when the cellular genome, wherein the virus is integrated, is a human genome, one may use the primers of SEQ ID NO :5 and/or 6.

In a particular embodiment of the invention, a purification step is provided between step (a) and step (b), so that only the amplified DNA of step(a) that has incorporated an Alu primer is subjected to the PCR of step (b).

For that purpose one can use for instance a biotinylated Alu primer during step (a), and then capture the amplified products that contain biotine by means of streptavidine, that has a high affinity for biotine. Other binding partners may be use, as will be recognized by one skilled in the art.

### The probe

In a preferred embodiment of the invention, the PCR of step (b) is performed in the presence of at least one probe that specifically hybridises with the viral sequence amplified, wherein the hybridisation of the probe allows for the detection and/or quantification of the integrated virus. A « detectable probe » means an oligonucleotide that is capable of emitting a signal, either directly or indirectly, through the use of various labels preferably onlywhen hybridised to its target sequence.

The probe can be labelled by any technique well known to those skilled in the art. The probe preferably carries a fluorescent moiety, *i.e.* chemicals which fluoresce when exposed to ultraviolet light. A number of fluorescent materials are known and be utilized as labels. These include, for example, fluorescein, rhodamine, auramine, Texas Red, AMCA Blue, and Lucifer Yellow.

Radiactive labels can be utilized too, using for instance isotopes such as ³H, ¹⁴C, ³²P, or ³⁵S.

Enzyme labels are likewise useful, and can be detected by any of the known colorimetric, spectrophotometric, fluorospectrophotometric, amperometric or gasometric techniques. The enzyme may be conjugated to the probe by reaction with bridging molecules such as carbodiimides, diisocyanates, or glutaraldehyde.

In a particular embodiment, it may be useful to use several different probes, *e.g.* two probes that specifically hybridise to the amplified end region of the virus (see Figure 1A, for example) and that may be labelled by labels of different types.

Detection of the signal emitted by the probe is preferably performed simultaneously with the amplification of the DNA according to step (b) of the method of the invention. One skilled in the art then usually refers to a "real-time" PCR.

In a particular preferred embodiment, one can use a hybridisation probe format, wherein two independent, single labelled probes hybridise adjacently on the amplicon internal to the flanking PCR primers. Advantageously the upstream probe is labelled at its 3'end with fluorescein (FL), and the downstream probe is labelled at its 5' end with LightCycler Red 640 (LC red 640), as described in Nitsche et al, 1999. After excitation by a light-emitting diode, a fluorescence resonance energy transfer (FRET) occurs from the FL (donor) to the LC Red (acceptor), increasing the LC Red signal, which is directly related to the amount of PCR product before the plateau is reached.

For HIV detection, one can advantageously use probes of SEQ ID NO :7 and SEQ ID NO: 8.

Alternatively, sequence detection systems may use the exonuclease assay, as described in Nitsche et al, 1999, or the molecular Beacon probeassay (Tyagi and Kramer, 1996; O'Doherty, 2002), wherein a fluorophore and a quencher moiety are attached to each terminus of the probing sequence.

### Kits

The invention further provides kits for performing the above method. A subject of the invention is thus a kit which comprises
- to carry out step(a) of the method, in a first container, or in separate containers, at least one primer for an *Alu* sequence and
   a primer for a viral sequence to be detected, wherein the primer for the viral sequence is linked to an anchor sequence that is not capable of hybridising to the viral genome nor to the cellular genome; and
- to carry out step(b) of the method, in another single container or in other separate containers, a primer for the viral sequence amplified in step (a), and a primer for the anchor sequence amplified in step (a).

The primers of the kits are as described above.

In a preferred embodiment the first container may contain two outward-facing primers for the *Alu* sequences.

The container or at least one of the containers intended for step (b) of the method, further comprises a detectable probe that specifically hybridises with the viral sequence to be amplified. Several different probes may be included in the kits if desired.

In a particular embodiment the kit comprises
- in a first container, two outward-facing primers for *Alu* sequences and an anchored primer for a HIV viral sequence, which anchored primer consists of SEQ ID NO:3; and
- in a second container, a primer of SEQ ID NO: 4, a primer of SEQ ID NO: 1, and probes of SEQ ID NO:7 and 8.

The probe of SEQ ID NO: 7 may preferably be modified with fluorescein at the 3' end; whereas the probe of SEQ ID NO:8 may preferably be modified with LC red 640 dye at the 5'end.

Advantageously at least one of the Alu primers may be biotinylated.

The kits may also contain means for performing the PCR amplication, i.e. a thermostable DNA polymerase, and dNTP solutions for instance.

The figures and examples illustrate the invention without limiting its scope.

### Description of the drawings:

Figure 1 is a schematic drawing that shows real-time PCR strategies and location of primers and probes for the quantification of integrated HIV-1 DNA (Fig. 1A), total HIV-1 DNA (Fig. 1B) and 2-LTR circle (Fig. 1C). Thin black arrows indicate primers and large black arrows indicate probes.

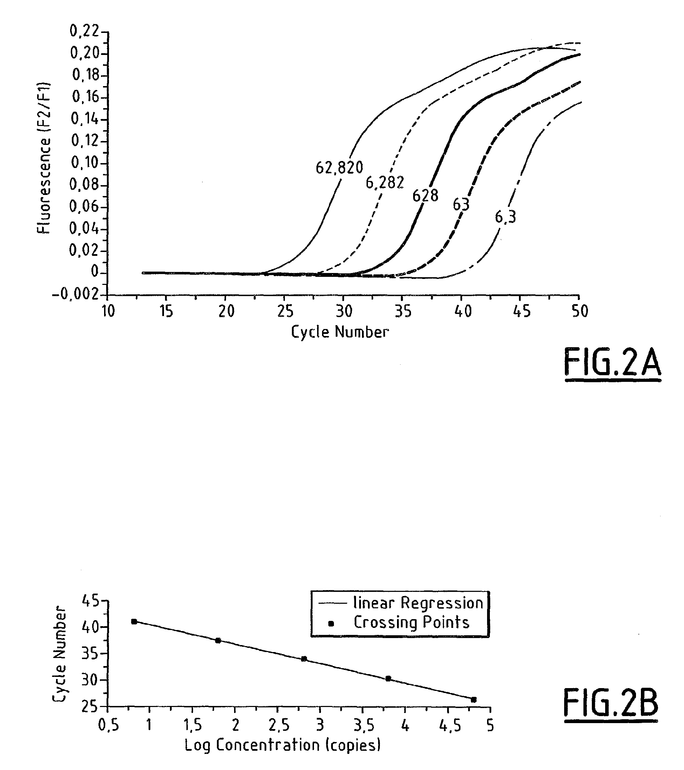

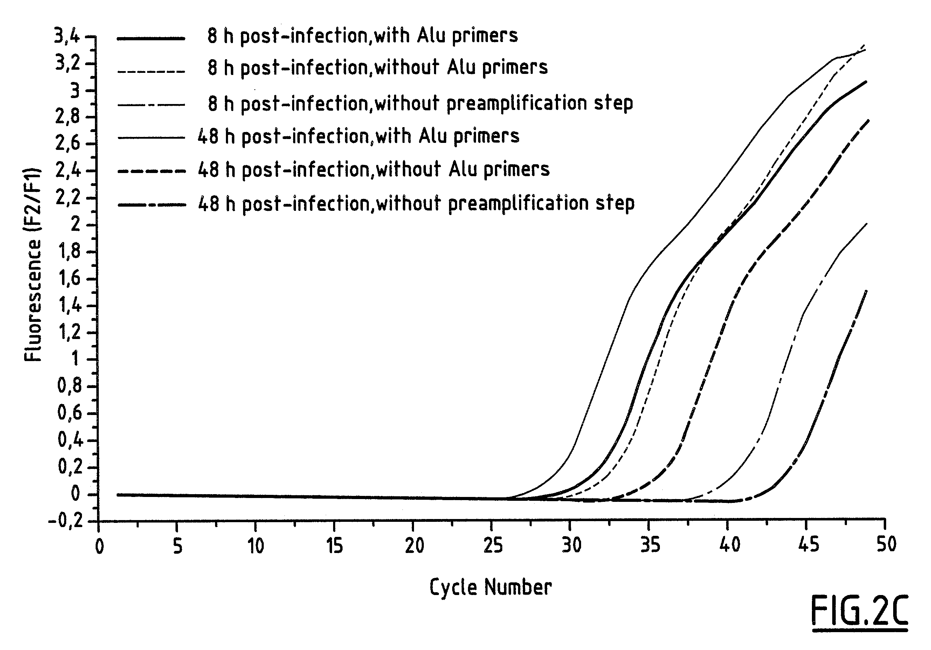

Figure 2 shows the characteristics of the *Alu*-LTR nested PCR. Fig. 2A : Fluorescence curves generated by two-step amplification of serial dilutions of HeLa R7 Neo cell DNA. The known copy numbers of each standard dilution are shown over the corresponding fluorescence curve. Fig. 2B : Linear regression for quantifying integrated HIV-1 DNA. Fig. 2C : Specificity of the *Alu*-LTR nested PCR. Fluorescence curves generated by amplification of viral DNA from CEM cells infected with the VSV-G pseudotyped HIV-1 R7 Neo virus and collected 8 h post-infection, in the presence of *Alu* primers in the absence of *Alu* primers or without preamplification step Fluorescence curves generated by amplification of viral DNA from CEM cells infected with the VSV-G pseudotyped HIV-1 R7 Neo virus and collected 48 h post-infection, in the presence of *Alu* primers in the absence of *Alu* primers or without preamplification step

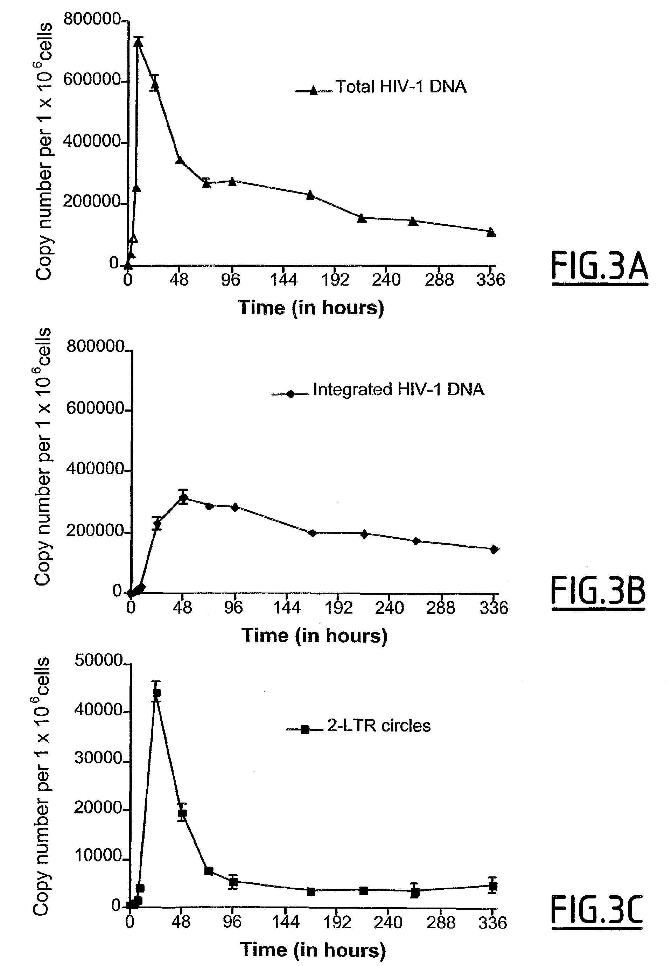

Figure 3: Quantification of total HIV-1 DNA (Fig. 3A), integrated HIV-1 DNA (Fig 3B) and 2-LTR circles (Fig 3C) during a single-round replication of the VSV-G pseudotyped HIV-1 R7 Neo virus into CEM cells. Values are means ± standard error of the mean. Depicted results are obtained from a representative experiment.

### Examples:

### Example 1: Quantification of integrated HIV

### 1.1. Generation of an integrated HIV-1 DNA standard

To accurately determine the integrated HIV-1 DNA copy number within a sample, the standard should be representative of a natural viral infection and contain numerous integration sites with a wide distribution of distances between the provirus LTR and the nearest *Alu* sequence. To generate a proper integrated HIV-1 DNA standard, HeLa cells were infected with a Δenv HIV-1 R7 Neo virus pseudotyped with the G glycoprotein from the Vesicular Stomatitis Virus (VSV-G). VSV-G pseudotyped HIV-1 R7 Neo virus stocks were prepared by cotransfection into 293T cells of the pR7 Neo Δenv vector with an expression vector encoding VSV-G. The pR7 Neo Δenv vector was constructed by deleting the envelope coding sequence of the HIV-1 R7 Neo genome (Feinberg *et al.,* 1991), in which a neomycin resistance gene replaces the HIV-1 nef coding sequence. Infected cells were then cultured for several weeks in the presence of G418 (500 mg/ml) to select cells that contained integrated viral DNA and to lose all unintegrated forms. Ten days upon initiation of the G418 selection, neomycin resistant cell clones were counted and numbered approximately at 5,000 clones. The standard cell line, designated as HeLa R7 Neo, contained a number of integrated viral DNA that matched with the total HIV-1 DNA copy number. Thus, based on total HIV-1 DNA and beta-globin quantifications (see below), the inventors estimated that the standard cell line contained 1.24 ± 0.03 proviruses per cell.

### 1.2. Alu-LTR- based real-time nested PCR procedure

Amplification reactions were performed using the Light Cycler Instrument (Roche Diagnostics, Meylan, France). In a first round of PCR, integrated HIV-1 sequences were amplified using two outward-facing *Alu* primers that anneal within conserved regions of the *Alu* repeat element together with an HIV-1 LTR-specific primer (Fig 1A). In addition, the inventors used an LTR primer extended with a lambda phage specific heel sequence at the 5' end of the oligonucleotide (L-M667) in this first amplification step (Fig. 1A). *Alu*-LTR sequences were amplified in duplicate from 1/50 of total cell DNA in a 20 ml reaction mixture comprising 1X Light Cycler Fast Start DNA Master Hybridization probes (Roche), 4 mM MgCl2, 100 nM of L-M667 primer and 300 nM of primers *Alu* 1 and *Alu* 2 (Sonza *et al.* 1996) (Table 1).

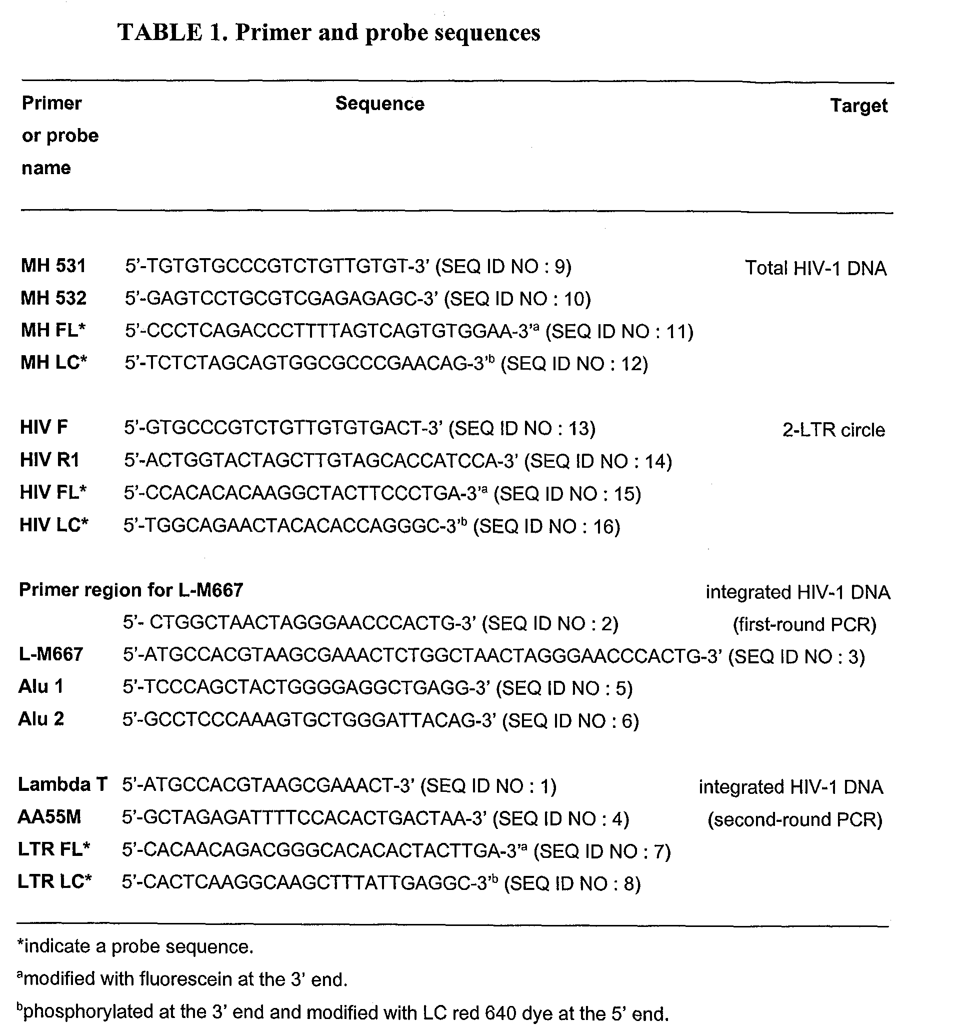

Given the high number of *Alu* elements within the human genome, abundant amplifications of inter-*Alu* sequences occurred simultaneously with the amplification of *Alu*-LTR sequences. To remain in the exponential phase, only 12 cycles of amplification were performed. Thus, the first-round PCR cycle conditions were as follows: a denaturation step of 8 min at 95 °C and then 12 cycles of amplification (95 °C 10 s, 60 °C 10 s , 72 °C 170 s).

In a second round of PCR, using the lambda specific primer (Lambda T) and a LTR-primer (AA55M), only products from the first-round PCR could be amplified (Fig. 1A). In contrast to the first-round PCR that generated fragments of varying lengths, the nested amplification resulted in discrete DNA fragments. Nested PCR was performed on 1/10 of the first-round PCR product in 1X Light Cycler Fast Start DNA Master Hybridization probes, 4 mM MgCl2, 300 nM of Lambda T primer, 300 nM of AA55M primer and 200 nM of each hybridization probe LTR FL and LTR LC (Table 1). Nested PCR cycling profile began with a denaturation step (95 °C for 8 min) followed by 50 cycles of amplification (95 °C 10 s, 60 °C 10 s , 72 °C 9 s).

The integrated HIV-1 DNA copy number was determined in reference to a standard curve generated by concomitant two-stage PCR amplification of serial dilution of the standard HeLa R7 Neo cell DNA (Fig. 2A) mixed with uninfected cell DNA to yield 50,000 cell equivalents. Quantification was achieved using the second derivative maximum method provided by the Light Cycler quantification software version 3.5 (Roche Diagnostics). In the present system, the linear regression obtained from amplification of serial dilutions of the integrated HIV-1 DNA standard was linear over a 5 log10 range and the present *Alu*-LTR nested PCR procedure allowed to detect ~ 6 proviruses within 50,000 cell equivalents (Fig. 2B).

### 1.3. Specificity of the Alu-LTR nested PCR assay

During the first-round PCR, L-M667 oligonucleotide can prime the formation of a single stranded DNA from all LTR containing HIV-1 DNA, leading to an overestimation of the actual integrated HIV-1 DNA copy number. To control these linear amplifications, the inventors performed a whole nested PCR procedure omitting or not *Alu* primers in the first-round PCR. Figure 2C displayed fluorescence curves obtained during amplification of cell DNA from CEM lymphoid cells infected with the VSV-G pseudotyped HIV-1 R7 Neo virus and collected 8 h or 48 h post-infection. In the presence of *Alu* primers, the fluorescence curves shifted, moderately but reproductively, to the left, when compared to an amplification performed in the absence of *Alu* primers (Fig. 2C). The shift was of about 1 cycle and 7 cycles, corresponding to a twofold and fifty-fold increase in copy numbers, for the 8 h and 48 h DNA time points, respectively. Furthermore, to determine the extent of second-round amplification of non-preamplified viral DNA, the inventors performed an additional control where the first-round PCR was replaced by a 4°C incubation step of the PCR mixture. As expected, the amplification curves shifted dramatically to the left when a preamplification step was performed. The shift corresponded to approximately a 2.3 log10 (9 cycles) and a 3.5 log10 (14 cycles) increase in copy numbers, for the 8 h and 48 h DNA time points respectively. Taken together, these findings indicate that PCR signal obtained in absence of *Alu* primers must be subtracted from the total signal, especially at early times following infection, and that the second-round amplification of non-preamplified viral DNA is efficiently prevented.

### 1.4. Synthesis and fate of HIV-1 DNA species in a single-round infection assay

To validate this integration assay, the inventors quantified integrated HIV-1 DNA, together with total HIV-1 DNA and 2-LTR circles in a single-round of viral replication. Eighteen millions of CEM cells were infected by VSV-G pseudotyped HIV-1 R7 Neo virus using 35 ng of p24gag antigen (measured by enzyme-linked immunosorbent assay, Perkin Elmer Life Sciences, Paris, France) per 1 x 106 cells. One hour post-infection, cells were washed in phosphate buffered saline (PBS), exposed to trypsin (25 mg/ml) for 1 min at 37 °C and washed once with medium and twice with PBS. The cells were then cultured in RPMI 1640 supplemented with 10% fetal calf serum. At each time point, 1 x 106 to 3 x 106 infected cells were collected. To eliminate residual pR7 Neo Δenv vector DNA, the sample collected were washed in PBS and incubated with 750 U to 1,500 U of DNase I (INVITROGEN, Cergy Pontoise, France) in a buffer comprising 20 mM Tris-Cl pH 8.3, 50 mM KCl, 2 mM MgCl2, for 1 h at room temperature. Cells were washed in PBS and dry cell pellets were frozen at - 80 °C until use. Total cell DNA was extracted using QIAamp blood DNA mini kit (Qiagen, Courtaboeuf, France).

Total HIV-1 DNA copy number was determined using previously described primers (Butler *et al.,* 2001) that annealed in the U5 region of the LTR (MH 531) and in the 5' end of the gag gene (MH 532) (Fig 1B). The 2-LTR circles were amplified with primers spanning the LTR-LTR junction (HIV F and HIV R1), as described by Brussel *et al.,* 2003 (Fig 1C). U5-gag sequences and 2-LTR junctions were amplified in duplicate from 1/50 of total cell DNA. Reaction mixtures contained 1X Light Cycler Fast Start DNA Master Hybridization probes (Roche Diagnostics), 4 mM MgCl2, 300 nM of forward and reverse primers, 200 nM of each fluorogenic hybridization probe, in a final volume of 20 ml. After an initial denaturation step (95 °C for 8 min), cycling profile for total HIV-1 DNA was: 50 cycles (95 °C 10 s, 60 °C 10 s, 72 °C 6 s) and for 2-LTR circles: 15 cycles (95 °C 10 s, 66 °C 10 s, 72 °C 10 s) followed by 35 cycles at the beginning of which the annealing temperature was decreased by 0.5 °C per cycle to the secondary target temperature (59 °C). The copy number of total HIV-1 DNA and 2-LTR circles were determined in reference to a standard curve prepared by amplification of quantities ranging from 10 to 1 x 105 copies of cloned DNA with matching sequences. The cell equivalents in sample DNA were calculated based on the amplification of the b globin gene (2 copies per diploid cell) using the Light Cycler Instrument and commercially available materials (Control kit DNA, Roche Diagnostics). The 2-LTR circle, total HIV-1 DNA and integrated HIV-1 DNA quantification results were expressed as copy number per 1 x 106 cells.

Viral DNA was detected by 3 h post-infection (p.i.). Total HIV-1 DNA level increased rapidly until 9 h p.i. to reach a maximum of 734,996 copies per 1 x 106 cells (Fig. 3A). Then total HIV-1 DNA level underwent a steep decrease till 72 h p.i., followed by a slow decay phase. In previous single-round infection assays, the initial steep decrease in total HIV-1 DNA level was reported to represent the proteasome-mediated degradation of unintegrated linear HIV-1 DNA (Butler *et al.,* 2002). The inventors detected integrated HIV-1 DNA by 3 h p.i., attesting for the rapid nuclear import of reverse transcription products and emphasizing the sensitivity of our integration assay. Integrated HIV-1 DNA level increased until 48 h p.i, to reach a maximum of 316,578 copies per 1 x 106 cells (Fig. 3B). By 72 h p.i., integrated HIV-1 DNA level matched with that of total HIV-1 DNA, as expected in a single-round of viral replication. This confirmed the accuracy of the quantification method of the invention.

The slow decay in integrated HIV-1 DNA level may reflect the death of infected cells and/or the slow division rate of infected cells carrying an integrated provirus compared to uninfected ones. Two-LTR circles, as detected by the presence of LTR-LTR junctions, were observed as early as 3 h p.i. Two-LTR circle level attained 44,253 copies per 1 x 106 cells by 24 h p.i. and declined thereafter to reach a quasi steady level from 96 h p.i. till the end of the culture (Fig. 3C). Two-LTR circles were shown to be stable DNA forms incapable of self-replication and consequently, they are diluted as a function of cell division (Butler *et al.,* 2002 ; Pierson *et al.,* 2002). Accordingly, a dilution effect resulting from cell division may account for the decrease in 2-LTR circle level observed before 96 h p.i. In contrast, steady LTR-LTR junction levels from 96 h p.i. till the end of the culture were unexpected. Nevertheless, the non-specific integration of some 2-LTR circles (less than 10% in this experiment) into the host cell genome might explain the stability of LTR-LTR junction levels. Integrated LTR-LTR junctions may have been transmitted from mother to daughter cells at each division leading to steady LTR-LTR junction levels in spite of cell proliferation. Likewise, it was previously shown that some linear HIV-1 DNA could be integrated into the cell genome by an integrase-independent process (Gaur *et al.,* 1998). This finding indicates that detection of LTR-LTR junctions does not necessarily attest the presence of unintegrated forms of HIV-1 DNA.

### Example 2: Quantification of integrated spumavirus

Integrated HFV (Human Foamy Virus) DNA quantification was performed by using an *Alu*-LTR nested PCR as described for amplification of HIV-1 integrated DNA, as described in Example 1. Briefly, in the first-round PCR, *Alu*-LTR sequences, derived from integrated viral genomes, were amplified in a 20 µl reaction mixture containing 1x Light Cycler Fast Start DNA Master Hybridation probes (Roche), 4 mM MgCl₂, 100 nM of formard primer Lambda T-SpA and 300 nM of primers Alu 1 and Alu 2. Nested PCR was performed on 1/10 of the first round PCR cycle PCR products in 1x Light Cycler Fast Start DNA Master Hybridation probes, 4 mM MgCl₂, 300 nM of forward primer Lambda T, 300 nM of primer Nested R and 200 nM of each hybridation probes Sp FL and Sp LC (Table 2).

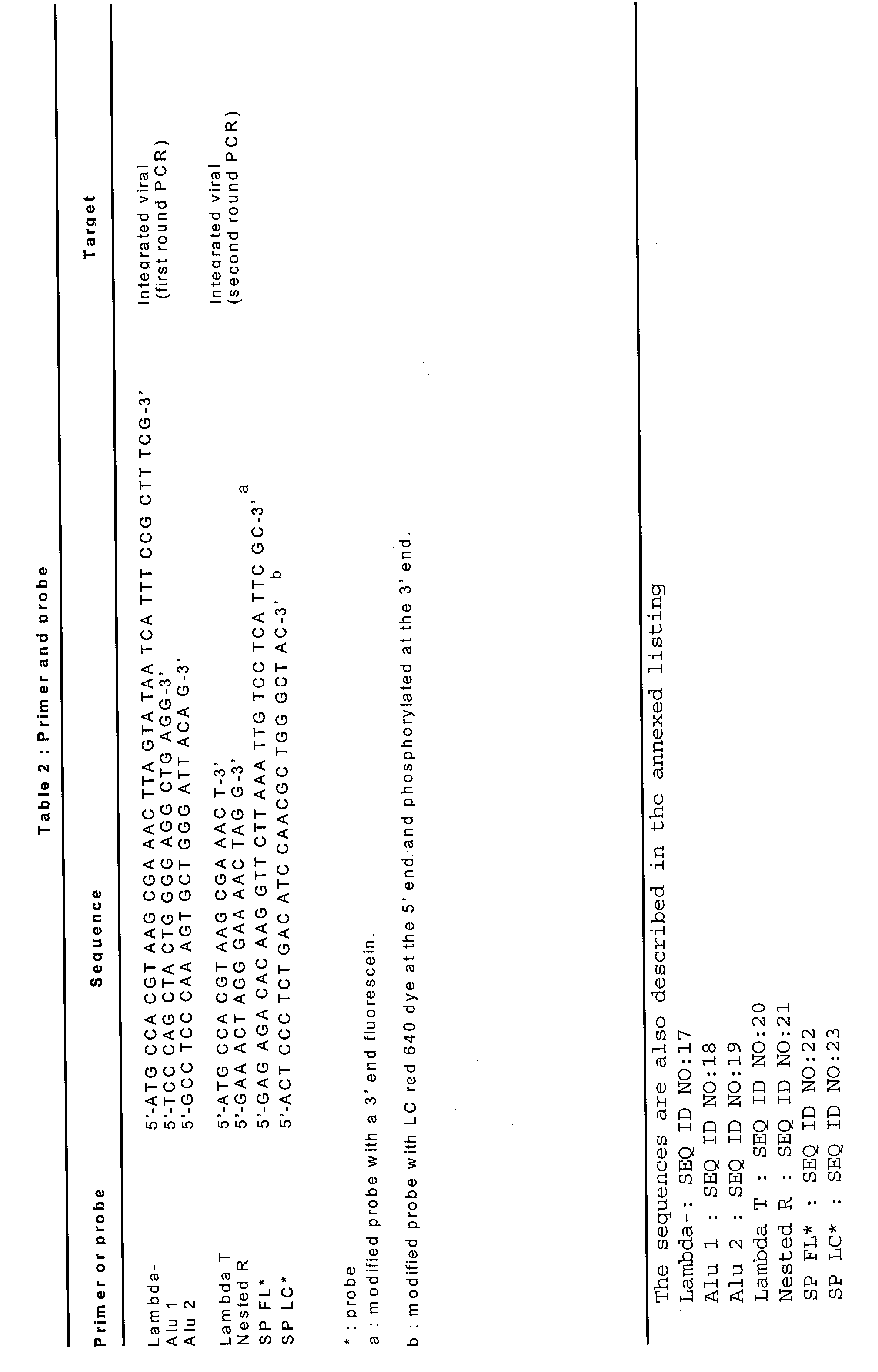

PCR cycle conditions are given in Table 3

**TABLE 3 :**

| PCR cycle conditions | | | |
|---|---|---|---|
| Target | RT | Denaturation | PCR cycles |
| Integrated viral DNA | - | 95°C 8min | (95°C 10s, 60°C 10s, 72°C |
| (first round PCR) | | | 170s)x15 |
| Integrated viral DNA | - | 95°C 8min | (95°C 10s, 63°C 10s, 72°C 8s)x50 |
| (second round PCR | | | |

Quantification of integrated HFV copies were determined in reference to a standard curve generated by the concomitant two-rounds PCR amplification of serial dilutions of the standard U3MG chronically infected cells. The invertors determined by serial dilutions of DNA and by Southern Blotting that viral DNA was exclusively present as integrated in cells. To control linear amplification arising from Lambda T-Sp A primer, the inventors performed, for each sample, a whole PCR protocol omitting *Alu* primers in the first round of PCR. Copy number of integrated DNA in each sample is determined by subtracting integrated DNA quantification in the absence of *Alu* primers from copy number measured in the presence of *Alu* primers. This *Alu*-LTR nested PCR procedure allowed to detect ~ 10 HFV proviruses within 50,000 cell equivalents.

### References

Britten, R. J., W. F. Baron, D. B. Stout, and E. H. Davidson. 1988. Sources and evolution of human Alu repeated sequences. Proc Natl Acad Sci U S A. 85:4770-4.

Brussel, A., D. Mathez, S. Broche-Pierre, R. Lancar, T. Calvez, P. Sonigo, and J. Leibowitch. 2003. Longitudinal monitoring of 2-long terminal repeat circles in peripheral blood mononuclear cells from patients with chronic HIV-1 infection. AIDS. 17:645-52.

Butler, S. L., M. S. Hansen, and F. D. Bushman. 2001. A quantitative assay for HIV DNA integration in vivo. Nat Med. 7:631-4.

Butler, S. L., E. P. Johnson, and F. D. Bushman. 2002. Human immunodeficiency virus cDNA metabolism: notable stability of two-long terminal repeat circles. J Virol. 76:3739-47.

Chun, T. W., L. Stuyver, S. B. Mizell, L. A. Ehler, J. A. Mican, M. Baseler, A. L. Lloyd, M. A. Nowak, and A. S. Fauci. 1997. Presence of an inducible HIV-1 latent reservoir during highly active antiretroviral therapy. Proc Natl Acad Sci U S A. 94:13193-7.

Feinberg, M. B., D. Baltimore, and A. D. Frankel. 1991. The role of Tat in the human immunodeficiency virus life cycle indicates a primary effect on transcriptional elongation. Proc Natl Acad Sci U S A. 88:4045-9.

Gaur, M., and A. D. Leavitt. 1998. Mutations in the human immunodeficiency virus type 1 integrase D,D(35)E motif do not eliminate provirus formation. J Virol. 72:4678-85.

Nitsche A., Schmidt C.A., Landt O, and Siegert W., 1999. Different real-time PCR formats compared for the quantitative detection of human cytomegalovirus DANN. Clinical Chemistry, 45:11, 1932-1937.

O'Doherty, U., W. J. Swiggard, D. Jeyakumar, D. McGain, and M. H. Malim. 2002. A sensitive, quantitative assay for human immunodeficiency virus type 1 integration. J Virol. 76:10942-50.

Pierson, T. C., T. L. Kieffer, C. T. Ruff, C. Buck, S. J. Gange, and R. F. Siliciano. 2002. Intrinsic Stability of Episomal Circles Formed during Human Immunodeficiency Virus Type 1 Replication. J Virol. 76:4138-44.

Sonza, S., A. Maerz, N. Deacon, J. Meanger, J. Mills, and S. Crowe. 1996. Human immunodeficiency virus type 1 replication is blocked prior to reverse transcription and integration in freshly isolated peripheral blood monocytes. J Virol. 70:3863-9.

Tyagi, S; and F.R. Kramer, 1996. Molecular beacons:probes that fluoresce upon hybridisation. Nat. Biotechnol. 14:303-308

Vandegraaff, N., R. Kumar, C. J. Burrell, and P. Li. 2001. Kinetics of human immunodeficiency virus type 1 (HIV) DNA integration in acutely infected cells as determined using a novel assay for detection of integrated HIV DNA. J Virol. 75:11253-60.

## Claims

1. An *in vitro* method of detecting and/or quantifying a virus that is integrated in a cellular genome, which method comprises the steps of:
(a) amplifying DNA of a biological sample with PCR using at least one primer for an *Alu* sequence and a primer for a viral sequence, wherein the primer for the viral sequence is linked to an anchor sequence that is not capable of hybridising to the viral genome nor to the cellular genome;
(b) subjecting the amplified DNA to another amplification with PCR using a primer for the viral sequence that was amplified in step (a), and a primer for the anchor sequence that was amplified in step (a);
(c) detecting and/or quantifying the DNA that was amplified through steps (a) and (b), and that is correlated with the number of copies of the virus integrated in the cellular genome.

2. The method of claim 1, wherein the virus is a retrovirus.

3. The method of claim 2, wherein the virus is HIV.

4. The method according to any of claims 1 or 2, wherein the anchor sequence is SEQ ID NO: 1.

5. The method according to any of claims 1 to 3, wherein the virus is a retrovirus and the primer for the viral sequence used in step (a) hybridises to an LTR sequence of the retrovirus.

6. The method according to claim 5, wherein the primer for the viral sequence used in step (a) hybridises to the R region of the 5'LTR of the retrovirus and the primer used in step (b), for the viral sequence amplified in step (a), is an antisense primer that hybridises to the U5 region of the 5'LTR of the retrovirus.

7. The method according to claim 6, wherein the primer for the viral sequence used in step (a) comprises SEQ ID NO:2.

8. The method according to any of claims 6 or 7, wherein the primer used in step (b), for the viral sequence amplified in step (a), consists of SEQ ID NO:4.

9. The method according to any of claims 1 to 8, wherein the PCR of step (a) employs two outward-facing primers for the *Alu* sequences.

10. The method according to any of claims 1 to 9, wherein the PCR of step (b) is performed in the presence of at least one detectable probe that specifically hybridises with the viral sequence amplified, wherein the hybridisation of the probe allows for the detection and/or quantification of the integrated virus.

11. The method of claim 10, wherein the probe carries a fluorescent moiety.

12. The method according to any of claims 1 to 11, wherein step (a) comprises from 10 to 15 cycles of PCR.

13. The method according to any of claims 1 to 12, wherein a ten-fold dilution is performed between step (a) and step (b).

14. The method according to any of claims 1 to 13, wherein a purification of the amplified DNA is performed between step (a) and step (b), so that the amplication of step (b) occurs only on the DNA that was amplified during step (a) and that has incorporated an Alu primer.

15. The method according to claim 1, which method comprises the steps of:
(a) amplifying DNA of a human biological sample with 12 cycles of PCR using two outward-facing primers for *Alu* sequences and a primer for a HIV viral sequence, wherein the primer for the viral sequence is SEQ ID NO:2, linked to an anchor sequence SEQ ID NO:1, so that the sequence of the anchored primer consists of SEQ ID NO:3;
(b) subjecting the amplified DNA to a ten-fold dilution followed by another amplification with PCR using a primer of SEQ ID NO: 4 for the viral sequence that was amplified in step (a), and a primer of SEQ ID NO: 1 for the anchor sequence that was amplified in step (a) ; in the presence of probes of SEQ ID NO:7 and 8;
(c) detecting and/or quantifying the DNA that was amplified through steps (a) and (b), and that is correlated with the number of copies of the HIV virus that is integrated in the human genome.

16. A kit for performing the method of claim 1 , which comprises
- to carry out step(a) of the method, in a first container, or in separate containers, at least one primer for an *Alu* sequence and a primer for a viral sequence to be detected, wherein the primer for the viral sequence is linked to an anchor sequence that is not capable of hybridising to the viral genome nor to the cellular genome; and
- to carry out step(b) of the method, in another single container or in other separate containers, a primer for the viral sequence amplified in step (a), and a primer for the anchor sequence amplified in step (a).

17. The kit according to claim 16, wherein the viral sequence is HIV.

18. The kit according to any of claims 16 or 17, wherein the anchor sequence is SEQ ID NO:1.

19. The kit according to any of claim 18, wherein the primer for the anchor sequence amplified in step (a) consists of SEQ ID NO:1.

20. The kit according to any of claims 16 to 19, wherein the primer for the viral sequence to be detected hybridises to a LTR sequence of the virus.

21. The kit according to any of claims 16 to 20, wherein the virus is HIV and the primer for the viral sequence to be detected hybridises to the R region of the 3'LTR of HIV and the primer used in step (b), for the viral sequence amplified in step (a), is an antisense primer that hybridises to the U5 region of the 3'LTR of the HIV.

22. The kit according to claim 21, wherein the primer for the viral sequence to be detected comprises SEQ ID NO:2.

23. The kit according to any of claims 21 to 22, wherein the primer used in step (b), for the viral sequence amplified in step (a), consists of SEQ ID NO:4.

24. The kit according to any of claims 16 to 23, wherein the first container contains two outward-facing primers for the Alu sequences.

25. The kit according to any of claims 16 to 24, wherein the container or at least one of the containers intended for step (b) of the method, further comprises a detectable probe that specifically hybridises with the viral sequence to be amplified.

26. The kit according to claim 25, wherein the probe carries a fluorescent moiety.

27. The kit according to claim 16, which comprises
- in a first container, two outward-facing primers for Alu sequences and an anchored primer for a HIV viral sequence, which anchored primer consists of SEQ ID NO:3; and
- in a second container, a primer of SEQ ID NO: 4, a primer of SEQ ID NO: 1, and probes of SEQ ID NO:7 and SEQ ID NO:8.

28. The kit according to claim 27, wherein the probe of SEQ ID NO: 7 is modified with fluorescein at the 3' end; and the probe of SEQ ID NO:8 is modified with LC red 640 dye at the 5'end.

29. The kit according to any of claims 16 to 28, wherein at least one of the Alu primers is biotinylated.

30. An *in vitro* method for monitoring the efficacy of a vaccine or of a therapeutic treatment, against a virus infection in a human or animal, which method comprises determining whether the virus is integrated in the cellular genome of the human or animal, by means of the detection and/or quantification method according to claim 1, wherein the fewer copies of integrated virus are detected in the cellular genome, the more efficient the vaccine or therapeutic treatment is.

31. An *in vitro* method for verifying the safety of an attenuated living viral vaccine in a human or animal, which method comprises determining whether the attenuated virus is integrated in the cellular genome of the human or animal, by means of the detection and/or quantification method according to claim 1, wherein the fewer copies of the attenuated virus are detected in the cellular genome, the safer the viral vaccine is.

32. An *in vitro* method for assessing the transfer efficacy of a viral vector useful in gene therapy in a human or animal, which method comprises detecting and/or quantifying the viral vector integrated in the cellular genome by means of the method according to claim 1, wherein the greater copies of the viral vector are detected in the cellular genome, the more efficient the transfer is.

33. An *in vitro* method for assessing the risk of cellular gene disruption caused by the transfer of a viral vector useful in gene therapy in a human or animal, which method comprises detecting and/or quantifying the viral vector integrated in the cellular genome of the human or animals by means of the method according to claim 1, wherein the fewer copies of the viral vector are detected in the cellular genome, the lower the risk of cellular gene disruption is.


SEQUENCE LISTING

<110>  INSERM

<120>  A quantification method for integrated viruses

<130>  BET 03P0846

<160>  23

<170>  PatentIn version 3.1

<210>  1
<211>  19
<212>  DNA
<213>  Artificial : oligonucleotide

<400>  1
atgccacgta agcgaaact                                                    19

<210>  2
<211>  23
<212>  DNA
<213>  Artificial : oligonucleotide

<400>  2
ctggctaact agggaaccca ctg                                               23

<210>  3
<211>  42
<212>  DNA
<213>  Artificial : oligonucleotide

<400>  3
atgccacgta agcgaaactc tggctaacta gggaacccac tg                          42

<210>  4
<211>  25
<212>  DNA
<213>  Artificial : oligonucleotide

<400>  4
gctagagatt ttccacactg actaa                                             25

<210>  5
<211>  24
<212>  DNA
<213>  Artificial : oligonucleotide

<400>  5
tcccagctac tggggaggct gagg                                              24

<210>  6
<211>  25
<212>  DNA
<213>  Artificial : oligonucleotide

<400>  6
gcctcccaaa gtgctgggat tacag                                              25


<210>  7
<211>  27
<212>  DNA
<213>  Artificial : oligonucleotide

<400>  7
cacaacagac gggcacacac tacttga                                            27


<210>  8
<211>  25
<212>  DNA
<213>  Artificial : oligonucleotide

<400>  8
cactcaaggc aagctttatt gaggc                                              25


<210>  9
<211>  20
<212>  DNA
<213>  Artificial : oligonucleotide

<400>  9
tgtgtgcccg tctgttgtgt                                                    20


<210>  10
<211>  20
<212>  DNA
<213>  Artificial : oligonucleotide

<400>  10
gagtcctgcg tcgagagagc                                                    20


<210>  11
<211>  28
<212>  DNA
<213>  Artificial : oligonucleotide

<400>  11
ccctcagacc cttttagtca gtgtggaa                                           28


<210>  12
<211>  24
<212>  DNA
<213>  Artificial : oligonucleotide

<400>  12
tctctagcag tggcgcccga acag                                               24


<210>  13
<211>  21

<212>  DNA
<213>  Artificial : oligonucleotide

<400>  13
gtgcccgtct gttgtgtgac t                                                21


<210>  14
<211>  27
<212>  DNA
<213>  Artificial : oligonucleotide

<400>  14
actggtacta gcttgtagca ccatcca                                          27


<210>  15
<211>  24
<212>  DNA
<213>  Artificial : oligonucleotide

<400>  15
ccacacacaa ggctacttcc ctga                                             24


<210>  16
<211>  22
<212>  DNA
<213>  Artificial : oligonucleotide

<400>  16
tggcagaact acacaccagg gc                                               22


<210>  17
<211>  42
<212>  DNA
<213>  Artificial : oligonucleotide

<400>  17
atgccacgta agcgaaactt agtataatca tttccgcttt cg                         42


<210>  18
<211>  24
<212>  DNA
<213>  Artificial : oligonucleotide

<400>  18
tcccagctac tggggaggct gagg                                             24


<210>  19
<211>  25
<212>  DNA
<213>  Artificial : oligonucleotide

<400>  19
gcctcccaaa gtgctgggat tacag                                            25

<210>  20
<211>  19
<212>  DNA
<213>  Artificial : oligonucleotide

<400>  20
atgccacgta agcgaaact                                                    19


<210>  21
<211>  19
<212>  DNA
<213>  Artificial : oligonucleotide

<400>  21
gaaactaggg aaaactagg                                                    19


<210>  22
<211>  35
<212>  DNA
<213>  Artificial : oligonucleotide

<400>  22
gagagacaca aggttcttaa attgtcctca ttcgc                                  35


<210>  23
<211>  29
<212>  DNA
<213>  Artificial : oligonucleotide

<400>  23
actccctctg acatccaacg ctgggctac                                         29